# EUROPEAN PATENT SPECIFICATION

(11) **EP 2 641 532 B1**
(45) Date of publication and mention of the grant of the patent: **22.04.2015**
(21) Application number: 12822154.6
(22) Date of filing: 14.05.2012
(51) Int. Cl.: A61B 1/12, A61B 1/00

(54) **ENDOSCOPE CLEANING/DISINFECTING APPARATUS**
ENDOSKOPREINIGUNGS/DESINFEKTIONSVORRICHTUNG
DISPOSITIF DE NETTOYAGE ET DE DESINFECTION D'ENDOSCOPES

(30) Priority: 09.08.2011 JP 2011174279
(43) Date of publication of application: 25.09.2013
(73) Proprietor: Olympus Medical Systems Corp., Tokyo 151-0072 (JP)
(72) Inventor: SUZUKI Eiri, Tokyo 151-0072 (JP); KOBAYASHI Kenichi, Tokyo 151-0072 (JP)
(74) Representative: Schicker, Silvia
(86) International application number: PCT/JP2012/062319
(87) International publication number: WO 2013/021701

(56) References cited:
- JP-A- 2007 202 860
- JP-A- 2009 165 507
- JP-A- 2009 226 193
- JP-A- 2010 029 467
- JP-A- 2010 029 467
- JP-A- 2011 139 777
- US-A1- 2010 071 736

## Description

### Technical Field

The present invention relates to an endoscope cleaning/disinfecting apparatus, and more particularly to an endoscope cleaning/disinfecting apparatus capable of reading information of an endoscope to be cleaned and disinfected.

### Background Art

In recent years, endoscopes have been widely used in a medical field and an industrial field. Endoscopes that are used in the medical field enable observation of organs in bodies by elongated insertion portions being inserted into the bodies, and various treatments with use of treatment instruments inserted into insertion channels for treatment instruments included in the endoscopes in accordance with necessity.

The endoscopes in the medical field are used by being inserted into bodies especially for the purpose of inspection and treatment, and therefore, need to be cleaned and disinfected in order to be used again after use. As a method for cleaning and disinfecting the used endoscopes, for example, a method which performs cleaning/disinfecting by using an endoscope cleaning/disinfecting apparatus (hereinafter, simply called a cleaning/disinfecting apparatus) is well known.

With use of a cleaning/disinfecting apparatus, cleaning, disinfecting, rinsing, draining and the like (hereinafter, called a cleaning/disinfecting process) can be automatically performed for an endoscope only by the endoscope being set into a cleaning/disinfecting tank of the cleaning/disinfecting apparatus. On this occasion, in the endoscope, a cleaning liquid and a disinfecting liquid are supplied to not only an outer surface of the endoscope, but also insides of a plurality of conduits such as a known air/water feeding conduit, a suction conduit, and a treatment instrument insertion conduit which the endoscope internally has, whereby interiors of the respective conduits in the endoscope are also cleaned, and disinfected by the disinfecting liquid.

For management of the cleaning/disinfecting treatment of an endoscope, for example, management of disinfection history, a cleaning/disinfecting apparatus is provided with an endoscope information reading section that reads information of an endoscope, for example, information of the kind of the endoscope. For example, an endoscope information reading section is provided at an upper panel of an apparatus main body.

Further, as disclosed in Japanese Patent Application Laid-Open Publication No. 2010-63610, the apparatus has been also proposed, which is provided with an endoscope information reading section in a cleaning tank, and can read information of an endoscope that is set in the cleaning tank.

However, in the case where the endoscope information reading section is provided on the upper panel of the apparatus main body, an operator is required to take an operation procedure of: moving a used endoscope close to the endoscope information reading section and causing the endoscope information reading section to read the endoscope information; and thereafter setting the endoscope in the cleaning tank. Since the endoscope comprises the elongated insertion section, the operator has to move the endoscope close to the endoscope information reading section while holding the elongated insertion section in a lump, and therefore there is a fear that a part of the endoscope may touch a surface of the apparatus main body. It is undesirable that an outer surface of the apparatus main body is contaminated by the touch of the endoscope which has not cleaned and disinfected.

Further, in the case of the cleaning/disinfecting apparatus in which the endoscope information reading section is provided in the cleaning tank, when housing a plurality of endoscopes and performing cleaning or the like of the plurality of endoscopes, there arises a problem of a case where reading of the endoscope information of a second endoscope and subsequent endoscopes is not securely performed because of hindrance of an endoscope already set in the cleaning tank.

Accordingly, an object of the present invention is to provide an endoscope cleaning/disinfecting apparatus in which a plurality of endoscopes can be set and which is capable of reliably reading information of an endoscope other than an already set endoscope without hindrance thereof.

US 2010/071736 A discloses a cleaning apparatus that can clean multiple endoscopes simultaneously. The apparatus comprises a combination authorization information memory unit that stores combination authorization information that specifies whether or not simultaneous cleaning in the cleaning apparatus is allowed for every combination of various types of endoscopes. A determination unit determines, referring to the combination authorization information stored in the combination authorization information memory unit, whether or not the combination of endoscopes set in the cleaning apparatus to be simultaneously cleaned falls into one of the allowed combinations.

### Disclosure of Invention

### Means for Solving the Problem

An endoscope cleaning/disinfecting apparatus according to claim 1 is provided.

### Brief Description of the Drawings

Fig. 1 is a perspective view of a cleaning/disinfecting apparatus according to an embodiment of the present invention;
Fig. 2 is a plan view of a cleaning/disinfecting apparatus 1 at a time of two endoscopes being accommodated and set in a cleaning tank 4, according to the embodiment of the present invention;
Fig. 3 is a perspective view of a top surface portion of the cleaning/disinfecting apparatus 1 at the time of two endoscopes being accommodated and set in the cleaning tank 4 seen from a front, according to the embodiment of the present invention;
Fig. 4 is a sectional view for explaining a configuration of a cleaning case 6 provided with an endoscope information reading section 64, according to the embodiment of the present invention;
Fig. 5 is a partial cross-sectional view showing a configuration of an endoscope information reading section formed by an antenna 65 being sandwiched between film-shaped resin members, according to a modification of the embodiment of the present invention; and
Fig. 6 is a view for explaining an example of configuring a concave portion accommodating an accessory by a part of the cleaning tank 4, and providing an endoscope information reading section at an accessory cleaning section having the concave portion, according to a modification of the embodiment of the present invention.

### Best Mode for Carrying Out the Invention

Hereinafter, an embodiment of the present invention will be described in detail with reference to the drawings.

### (Entire configuration of a cleaning/disinfecting apparatus)

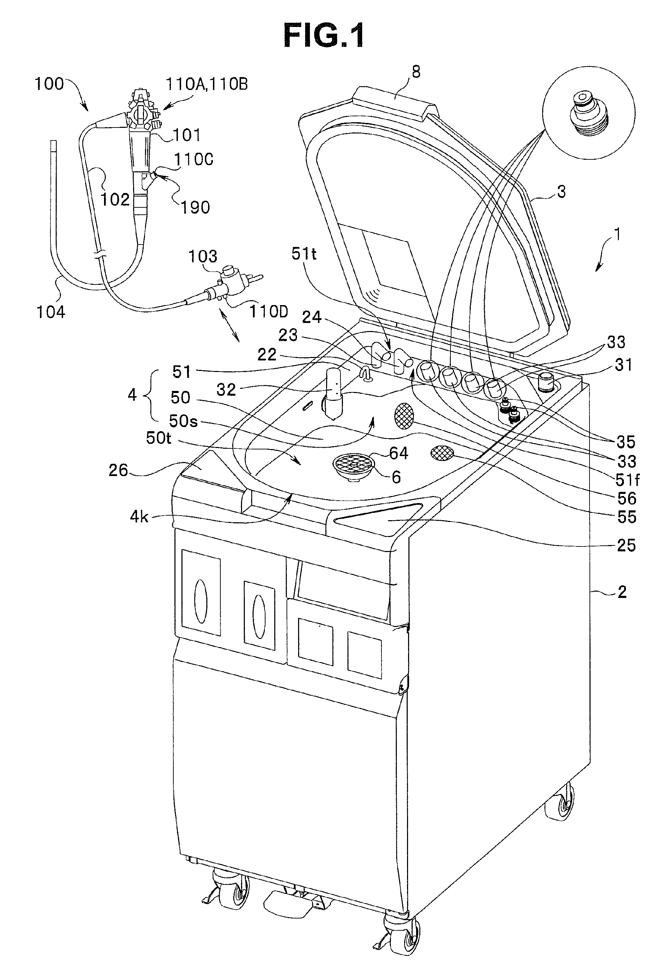

Fig. 1 is a perspective view of a cleaning/disinfecting apparatus according to the embodiment of the present invention. Fig. 1 shows a state in which a top cover of the cleaning/disinfecting apparatus is opened, and an endoscope 100 can be housed in a cleaning/disinfecting tank 4.

As shown in Fig. 1, the cleaning/disinfecting apparatus 1 is an apparatus for cleaning and disinfecting the endoscope 100 which is used, and a main part is configured by an apparatus main body 2, and a top cover 3 that is connected to a top portion thereof to be openable and closable via, for example, a hinge not illustrated.

When the top cover 3 is in a state in which the top cover is closed to the apparatus main body 2, the top cover 3 is configured to be fixed to the apparatus main body 2 by, for example, a latch 8 that is arranged in a position where the apparatus main body 2 and the top cover 3 are opposed to each other. A plurality of endoscopes to be cleaned are accommodated in the cleaning/disinfecting tank (hereinafter, called a cleaning tank) 4 at a top portion of the apparatus main body 2, and are cleaned and disinfected. The endoscope 100 usually includes an operation section 101, a universal cord 102, a connector 103 and an insertion portion 104.

In an inside of the apparatus main body 2, various tanks that store various liquids such as a cleaning liquid and a disinfecting liquid, various pumps for supplying the various liquids from the various tanks to the cleaning tank 4 and conduits in insides of the endoscope 100, a control section that controls the various pumps and various valves in accordance with respective process steps of a cleaning/disinfecting process, and the like are mounted. The control section also controls an endoscope information reading section that reads information of the endoscope 100.

Further, a main operation panel 25 on which setting switches such as a start switch of a cleaning and disinfecting operation of the apparatus main body 2 and a cleaning and disinfecting mode selection switch, and a display device such as a liquid crystal display device are arranged is provided near to, for example, a right end of a front surface side which an operator approaches, on the top surface of the apparatus main body 2.

Furthermore, a user ID reading section 26 that reads identification information (hereinafter, a user ID) of the operator is provided near to, for example, a left end of the front surface side which the operator approaches, on the top surface of the apparatus main body 2. The user ID reading section 26 reads the ID of a user by the user bringing an ID card of the user himself or herself close to the user ID reading section 26. The user ID which is read is used for management of cleaning/disinfecting treatment of an endoscope.

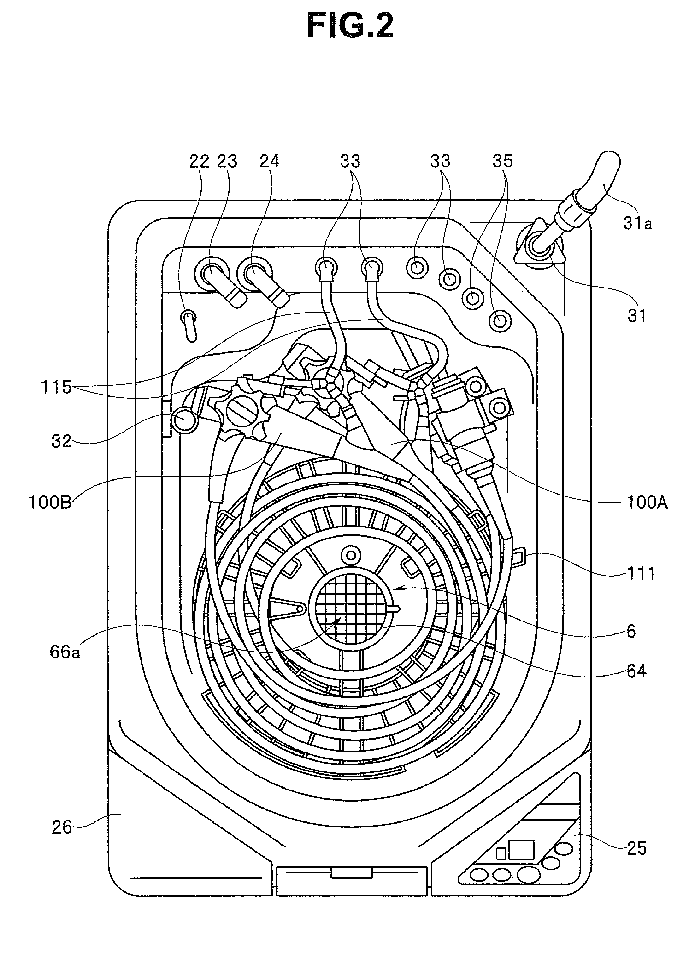

Further, a water supply hose connection port 31 to which a water supply hose 31a (see Fig. 2) that is connected to a city water faucet not illustrated for supplying city water to the apparatus main body 2 is connected is arranged at a rear surface side which is opposed to a front surface which the operator approaches, and is on the top surface of the apparatus main body 2.

The cleaning tank 4 is provided in a substantially central portion of the top surface of the apparatus main body 2, and can house a plurality of endoscopes 100. The cleaning tank 4 is made of stainless steel. The cleaning tank 4 is configured by a tank main body 50 and a terrace portion 51 that is peripherally provided continuously to an outer perimeter edge of an endoscope housing port of the tank main body 50.

When cleaning and disinfecting of the endoscope 100 is performed, the endoscope 100 is housed and set in the tank main body 50 in such a manner that respective portions of the endoscope 100 are locked at a holding net 111 (Fig. 2). A water drain pot 55 for draining liquids such as a cleaning liquid, water, alcohol and a disinfecting liquid that are supplied to the tank main body 50 from the tank main body 50 is provided at a bottom surface 50t of the tank main body 50.

Further, a circulation port 56 for supplying the water, the cleaning liquid, the disinfecting liquid and the like supplied to the tank main body 50 to respective conduits included in the inside of the endoscope 100 via means that will be described later, or for resupplying the cleaning liquid, the water, the disinfecting liquid and the like supplied to the tank main body 50 to the tank main body 50 again via a mesh filter or the like from a water supply circulation nozzle 24 which will be described later is provided, on a side surface 50s of an inner wall of the tank main body 50.

In the tank main body 50 of the cleaning tank 4, an ultrasound transducer and a heater not illustrated are further arranged. The ultrasound transducer gives vibration to the cleaning water or city water stored in the cleaning tank 4, and performs ultrasound cleaning or rinsing of an outer surface of the endoscope 100. Further, the heater is for heating the disinfecting liquid, city water and the like stored in the cleaning tank 4 to a predetermined temperature.

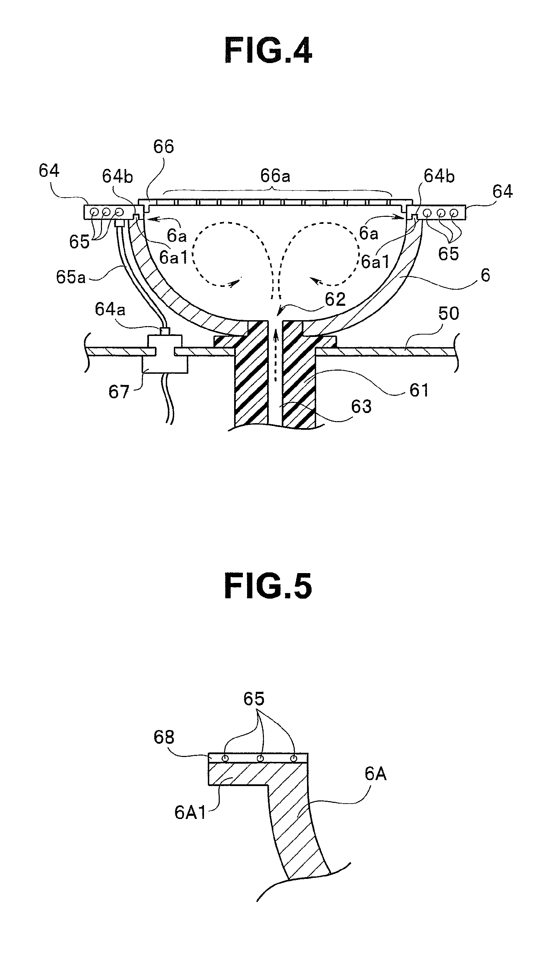

An accessory cleaning case (hereinafter, called a cleaning case) 6 is arranged detachably in a substantially central portion of the bottom surface 50t of the tank main body 50. In the cleaning case 6, accessories of the endoscope such as buttons such as respective scope switches of the endoscope 100, and dismountable components provided to be attached to the endoscope 100 are accommodated, and the respective accessories are cleaned and disinfected together with the endoscope 100. Namely, the cleaning case 6 configures an accessory cleaning section that cleans the accessories of the endoscope. A lid 66 (Fig. 4) having a lattice-shaped net is detachably provided on a top surface of the cleaning case 6, and by opening and closing of the lid 66, housing and removal of the accessories to be cleaned are enabled.

Furthermore, on the top surface of the cleaning case 6, an endoscope information reading section 64 for reading the information of the endoscope to be cleaned and disinfected, for example, information of the kind of the endoscope is provided. For example, in the operation section 101 of the endoscope 100, an IC tag for RFID (radio frequency identification system), that is, an RFID tag is embedded. The endoscope information reading section 64 includes an antenna for reading the information of the endoscope 100 by communicating with the RFID tag by radio when the operator brings the operation section 101 close to the endoscope information reading section 64.

A water level sensor 32 with a cover that detects levels of the cleaning liquid, the water, the disinfecting liquid and the like which are supplied to the tank main body 50 is provided at the side surface 50s of the tank main body 50.

On a surface other than a terrace surface 51t and a surface 51f of the terrace portion 51, that is, a surface parallel with the bottom surface 50t of the tank main body 50, a cleaning agent nozzle 22 for supplying a cleaning agent to the cleaning tank 4 by a cleaning agent pump that will be described later from a cleaning agent tank, and a disinfecting liquid nozzle 23 for supplying the disinfecting liquid to the cleaning tank 4 by a chemical pump from a chemical tank are arranged.

Furthermore, on the surface parallel with the bottom surface 50t of the tank main body 50 of the terrace portion 51, a water supply circulation nozzle 24 for supplying water to the cleaning tank 4 or for supplying the cleaning liquid, the water, the disinfecting liquid and the like which are sucked from the circulation port 56 of the tank main body 50 to the cleaning tank 4 again is arranged.

Further, on the surface 51f at a side opposed to an operator approaching position 4k of the terrace surface 51t of the terrace portion 51, a plurality of, in this case, four ports 33 for supplying water, a cleaning liquid, alcohol, a disinfecting liquid, air or the like to a plurality of conduits included in the inside of the endoscope 100, and water leakage detecting ports 35 are arranged. The four ports 33 are connector portions for supplying the cleaning liquid and the like to various conduits in the endoscope 100, for example, a suction conduit, an auxiliary water feeding conduit, an air/water feeding conduit, a forceps port and the like.

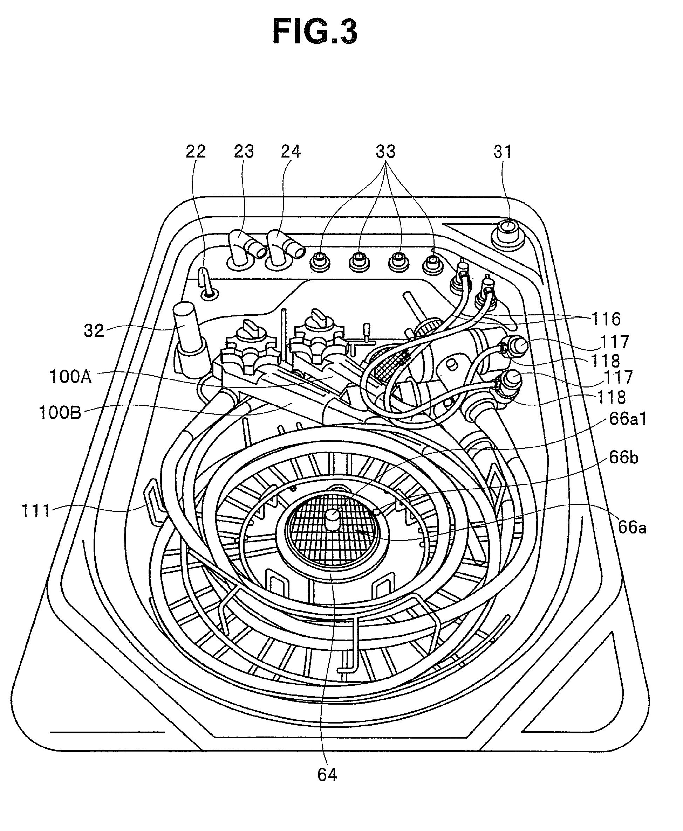

One ends of endoscope cleaning/disinfecting apparatus connection tubes (hereinafter, called cleaning connection tubes) 115 (Fig. 2, Fig. 3) are connected to the four ports 33, and the other ends of the cleaning connection tubes 115 are connected to corresponding pipe sleeves 110A to 110D of the endoscope 100 including the operation section 101, the insertion portion 104, the universal cord 102 and the connector 103.

The pipe sleeve 110A is for the suction conduit. The pipe sleeve 110B is for the air/water feeding conduit. The pipe sleeve 110C is for the forceps port. The pipe sleeve 110D is for the auxiliary water feeding conduit. The respective pipe sleeves communicate with the various conduits in the endoscope 100.

Fig. 2 is a plan view of the cleaning/disinfecting apparatus 1 at a time of two endoscopes being accommodated and set in the cleaning tank 4. Fig. 3 is a perspective view of a top surface portion of the cleaning/disinfecting apparatus 1 at the time of the two endoscopes being accommodated and set in the cleaning tank 4, seen from a front. Here, two endoscopes 100A and 100B are set in the cleaning tank 4, and the respective ports 33 are connected to pipe sleeves of various conduits of the two endoscopes 100A and 100B via the cleaning connection tubes 115. Note that Fig. 2 shows a state in which the cleaning/disinfecting tubes 115 are connected to the pipe sleeves 110A of the suction conduits and the pipe sleeves 110C of the forceps ports of the respective endoscopes 100A and 100B, and Fig. 3 shows a state in which water leakage detecting tubes 116 are connected to water leakage detecting connectors 118 attached to waterproof caps 117 attached to the connectors 103 of the two endoscopes.

The two endoscopes 100A and 100B are set in the cleaning tank 4 by using the holding net 111 detachable with respect to the cleaning tank 4 as shown in Fig. 2. The holding net 111 is a holding member formed by a rod member of a metal being folded, and is placed on the cleaning tank 4. When the endoscopes 100A and 100B are accommodated in such a manner as to be caught by concave and convex portions, which are formed by being folded, of the holding net 111, clearances are formed between the endoscopes 100A and 100B and the cleaning tank 4, and between the two endoscopes 100A and 100B, and the two endoscopes 100A and 100B are housed in predetermined positions or postures in the cleaning tank 4. As a result, portions at which the endoscopes 100A and 100B contact with each other tightly in close adhesion are not present, and therefore, the disinfecting liquid and the like are in contact with entire surfaces of outer surfaces of the endoscopes 100A and 100B, and portions which are not cleaned and disinfected are not present.

Namely, the holding net 111 configures a position defining section that defines positions or postures of the respective portions of the two endoscopes which are housed and set in the cleaning tank 4.

Note that the holding net 111 may be detachable from the cleaning tank 4, or may be fixed to the cleaning tank. Furthermore, the position defining section may be configured by a plurality of protruded portions provided on the bottom surface, the side surface and the like of the cleaning tank 4.

### (Configurations of the cleaning case 6 and the endoscope information reading section 64)

Fig. 4 is a sectional view for explaining a configuration of the cleaning case 6 provided with the endoscope information reading section 64. The cleaning case 6 is detachably fixed to the tank main body 50 of the cleaning tank 4. A fixing member 61 is fixed to the cleaning tank 4, and the cleaning case 6 is detachable with respect to the fixing member 61. The cleaning case 6 is made of stainless steel.

The cleaning case 6 disposed in the substantially central portion of the bottom surface 50t of the tank main body 50 has a cup shape, and is screwed onto the fixing member 61 and fixed thereto. An operator grasps and rotates the cleaning case 6, and thereby can detach or attach the cleaning case 6 from or to the cleaning tank 4. Note that the cleaning case 6 and the fixing member 61 may be attached by simple fitting without being screwed. Further, a space between the fixing member 61 and the tank main body 50 is sealed by a sealing member (not illustrated) so that the liquids do not leak therefrom.

An opening 62 is provided at a bottom portion of the cleaning case 6, and the opening 62 communicates with a case conduit in the apparatus main body 2 via a conduit 63 provided in the fixing member 61. From the case conduit, a liquid such as the disinfecting liquid is supplied from the pump in the inside of the apparatus main body 2, and from the opening 62 of the fixing member 61, the liquid spouts into the cleaning case 6. As shown by the dotted line in Fig. 4, the liquid supplied into the cleaning case 6 collides against the housed accessories, and cleaning and disinfecting of the accessories of the endoscopes are performed. While the liquid that spouts into the cleaning case 6 is agitated, a part of the liquid is released into the cleaning tank 4 from holes of a lattice-shaped net portion 66a of a lid 66.

The endoscope information reading section 64 is provided at an edge portion 6a at an upper side of the cup-shaped cleaning case 6. The endoscope information reading section 64 is a rigid ring-shaped member of a resin, and has a conductor wire of the antenna 65 for RFID embedded in an inside thereof. The resin is, for example, polypropylene or the like. A conductive member that configures the antenna 65 is disposed along the ring-shaped endoscope information reading section 64, in such a manner as to draw multiple circles in a spiral shape of drawing with a single stroke, or in such a manner as to draw a multiple circular arcs by drawing with a single stroke, when the cleaning case 6 is seen in plan view from the top surface.

The cleaning case 6 is of a metal of stainless steel, and therefore, the ultrasound cleaning force by the ultrasound transducer provided at the bottom portion of the cleaning tank 4 is not reduced.

A ring-shaped convex portion 6a1 is formed at the edge portion 6a of the upper side of the cleaning case 6, and a ring-shaped concave portion 64b is formed at a lower side of the endoscope information reading section 64 to be fitted onto the convex portion 6a1. The convex portion 6a1 and the concave portion 64b are fitted to each other, and by an adhesive, the endoscope information reading section 64 is fixed to the cleaning case 6.

The lid 66 is detachably fitted onto an inner circumferential side of the ring-shaped endoscope information reading section 64. The lid 66 is circular when the lid 66 is seen from the upper side, and has the lattice-shaped net portion 66a on an inner side of a circular frame, as shown in Fig. 2. Note that as shown in Fig. 3, the lid 66 is provided with a grasping portion 66a1, and the lid 66 is connected to the cleaning case 6 by a chain 66b for prevention of loss.

A wiring cable 65a connected to the antenna 65 includes a plurality of conducting wires covered with an insulation member, and is connected to a connector 67 provided at the bottom portion of the cleaning tank 4, by a connector 64a. Connection portions of the connectors 64a and 67 are sealed with a seal member not illustrated. The conductive wires of the wiring cable 65a of the antenna 65 are connected to a communication circuit for RFID.

Note that wiring is provided at the fixing member 61, and the wiring 65a of the antenna 65 may be connected to the communication circuit via a part of the fixing member 61 instead of the connector 67.

As shown in Fig. 2 and Fig. 3, in the state in which the two endoscopes 100A and 100B are set in the cleaning tank 4 in positions defined by the holding net 111, the endoscope information reading section 64 is not covered with the two endoscopes 100A and 100B, but is disposed in a site of the cleaning case 6 exposed toward the upper side of the cleaning tank 4. In other words, the site in which the cleaning case 6 that cleans the accessories of the endoscopes is disposed is the site that is exposed toward the upper side of the cleaning tank 4 even when a plurality of endoscopes are set in the cleaning tank 4, and the endoscope information reading section 64 is arranged at the site in the cleaning tank 4.

Further, as shown in Fig. 2 and Fig. 3, a plurality of (two in Fig. 2 and Fig. 3) endoscopes are disposed to surround the central portion of the cleaning tank 4 by the holding net 111 that is the position defining section, and the site which is exposed toward the upper side of the cleaning tank 4 is located in the central portion, and is located at the upper side apart from the bottom surface of the cleaning tank 4. The endoscope information reading section 64 is provided at the cleaning case 6 that is disposed in the central portion.

### (Operation)

Next, an operation of the cleaning/disinfecting apparatus 1 of the configuration as above will be described.

Hereinafter, a case in which the two endoscopes 100A and 100B are cleaned and disinfected will be described.

When an operator uses the cleaning/disinfecting apparatus 1, the operator performs a predetermined actuation operation to the main operation panel 25, and thereafter, brings an ID card of himself or herself close to the user ID reading section 26 to cause the cleaning/disinfecting apparatus 1 to read identification information of himself or herself.

Subsequently, the operator sets the used endoscope 100A in the cleaning/disinfecting apparatus 1, and first of all, the operator brings the portion containing an RFID tag of the endoscope 100A, in this case, the operation section 101, close to the endoscope information reading section 64 provided in the central portion of the cleaning tank 4.

The endoscope information reading section 64 communicates with the RFID tag of the endoscope 100A, and can read the information, for example, information of the kind of the endoscope 100A. The information of the endoscope that is read is supplied to the control section not illustrated in the apparatus main body 2. Note that in order to inform the operator that the information of the endoscope 100A is read by the endoscope information reading section 64, the control section of the cleaning/disinfecting apparatus 1 may perform control to produce a predetermined sound from a speaker, perform display expressing a success in reading on a display section of the main operation panel 25, or perform any one of or both of sound production and display.

After the operator causes the information of the endoscope of the endoscope 100A to be read, the operator sets the endoscope 100A in the holding net 111. As described above, the holding net 111 defines the positions of the respective portions of the endoscope 100A to be set, or the posture when the endoscope 100A is set. In the case of Fig. 2 and Fig. 3, when the endoscope 100A is set in the holding net 111, the insertion portion 101 and the universal cable 102 which respectively have elongated portions are disposed in such a manner as to surround the cleaning case 6 which is provided in substantially the center of the cleaning tank 4.

Accordingly, the cleaning case 6 is not covered with the endoscope 100A even after the endoscope 100A is set in the cleaning tank 4.

Next, the operator brings the portion containing the RFID tag of the endoscope 100B, in this case, the operation section 101, close to the endoscope information reading section 64 provided in the central portion of the cleaning tank 4. At this time, the endoscope information reading section 64 is not in the shadow of the endoscope 100A which is already set, and is exposed to the operator, and therefore, the operator can cause the cleaning/disinfecting apparatus 1 to read the information of the endoscope 100B reliably by bringing the operation section 101 including the RFID tag of the endoscope 100B close to the endoscope information reading section 64.

In other words, the endoscope information reading section 64 is arranged in the site which is exposed toward the upper side of the cleaning tank 4 without being covered with the endoscope 100A, in the state in which the endoscope 100A is set in the cleaning tank 4 in the position defined by the holding net 111, and therefore, the operator can bring the operation section 101 of the endoscope 100B which is to be set next close to the endoscope information reading section 64 to cause the endoscope information reading section 64 to read the endoscope information.

Conventionally, the endoscope information reading section is in the shadow of the endoscope which is already set, and the endoscope to be set next cannot be brought close to the endoscope information reading section in some cases. However, according to the present embodiment, the endoscope information reading section 64 is provided on the cleaning case 6 disposed in substantially the center of the cleaning tank 4, and further, the positions or the postures of the respective portions of the endoscope at the time of the endoscope being set in the cleaning tank 4 are defined by the holding net 111. Therefore, even if the endoscope which is already set in the cleaning tank 4 is present, the endoscope information reading section 64 is exposed from the cleaning tank 4 without being hindered by the endoscope which is set, and therefore, the operator can bring the endoscope to be set next to the endoscope information reading section 64 disposed in the exposed position, and can causes the endoscope information reading section 64 to read the information of the endoscope reliably.

The operator sets the endoscope 100B in the holding net 111 after the operator causes the information of the endoscope of the endoscope 100B to be read. The operator sets the two endoscopes 100A and 100B in the cleaning tank 4, and connects the cleaning connection tubes 115 for cleaning and disinfecting the respective conduits of the inside of the respective endoscopes to the ports 33 and the respective pipe sleeves. Subsequently, when the operator closes the top cover 3, and performs a predetermined operation to the main operation panel 25, the cleaning/disinfecting apparatus 1 starts a cleaning/disinfecting process.

As above, according to the aforementioned cleaning/disinfecting apparatus, the endoscope cleaning/disinfecting apparatus in which a plurality of endoscopes can be set, and which is capable of reliably reading the information of the next endoscope without the endoscope already set becoming a hindrance can be realized.

Further, the cleaning case 6 is detachable with respect to the apparatus main body 2, and therefore, repair and maintenance of the antenna wire of the endoscope information reading section 64 can be easily performed.

Note that the endoscope information reading section 64 may be configured to be detachable with respect to the cleaning case 6 so that repair and maintenance of the antenna wire of the endoscope information reading section 64 can be easily performed.

Furthermore, in the aforementioned endoscope information reading section 64, the antenna 65 is embedded in the rigid resin ring shape, but the endoscope information reading section may be formed so as to sandwich the antenna between film-shaped resin members. Fig. 5 is a partial cross-sectional view showing a configuration of the endoscope information reading section formed by sandwiching the antenna 65 between the film-shaped resin members, according to a modification of the present embodiment.

As shown in Fig. 5, a flange portion 6A1 is formed at an upper portion of a cleaning case 6A, and onto the flange portion 6A1, an endoscope information reading section 64A provided with the antenna 65 in a film-shaped resin member 68 is stuck by an adhesive or the like.

According to the endoscope information reading section 64A of the configuration as above, the same effect as in Fig. 4 can be obtained.

Furthermore, the endoscope information reading section may be provided at the accessory cleaning section configured by a part of the cleaning tank 4.

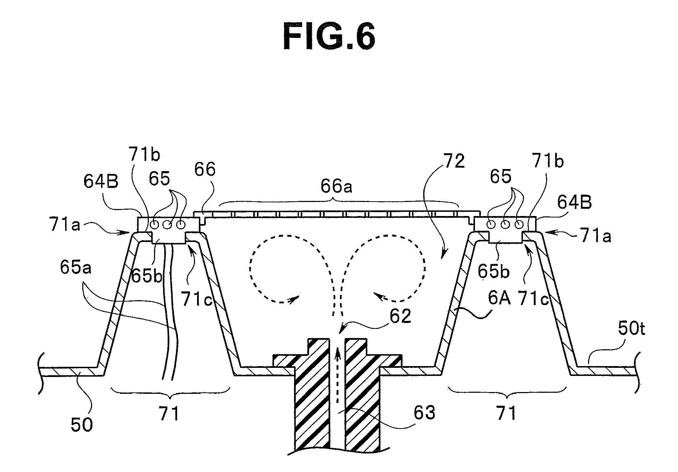

Fig. 6 is a view for explaining an example in which the concave portion which accommodates accessories is configured by a part of the cleaning tank 4, and the endoscope information reading section is provided in the accessory cleaning section having the concave portion.

As shown in Fig. 6, part of the cleaning tank main body 50 is molded to be protruded in a cylindrical shape, and a concave portion 72 surrounded by a protruded portion 71 protruded in a cylindrical shape from the bottom surface 50t is formed in the cleaning tank main body 50. As a result, the protruded portion 71 and the concave portion 72 configure the accessory cleaning section having a function equivalent to the cleaning case 6 described above.

A top portion 7 1 a of the ring-shaped protruded portion 71 has a planar portion 71b on which a ring-shaped endoscope information reading section 64B is mounted. A space between the endoscope reading section 64B and the planar portion 71b is sealed by a seal member not illustrated, and the ring-shaped endoscope reading section 64B is attached onto the planar portion 71b.

The ring-shaped endoscope information reading section 64B is a resin member, and the conductive wires of the antenna 65 are embedded in an inside thereof.

When the cleaning tank 4 is seen in plan view, holes 7 1 c are provided at a plurality of spots in the planar portion 71b which looks ring-shaped, and protruded portions 65b are provided at a plurality of spots in positions corresponding to the holes 71c. The holes 71 c and the protruded portions 65b are fitted to one another, whereby the endoscope information reading section 64B can be positioned to and attached onto the top portion 71a of the ring-shaped protruded portion 71. Further, from the protruded portions 65b, the conductive wires of the antenna 65 are led out.

Accordingly, in Fig. 6, the endoscope information reading section 64B is not covered with the endoscope already set either, and is also provided at an accessory cleaning section disposed at a site exposed toward the upper side of the cleaning tank 4.

Therefore, the protruded portion 71 and the concave portion 72 according to the configuration of Fig. 6 have a function equivalent to the cleaning case 6 of Fig. 4, and the endoscope information reading section 64B provided on the top portion 71a around the concave portion 72 can reliably read information of a plurality of endoscopes.

Note that in the example of Fig. 6, the endoscope information reading section 64B may be replaced with the endoscope information reading section 64A formed by the antenna 65 sandwiched between the film-shaped resin members shown in Fig. 5.

As above, according to the embodiment and the respective modifications described above, the endoscope cleaning/disinfecting apparatus in which a plurality of endoscopes can be set, and which can reliably read the information of the next endoscope without the endoscope already set becoming a hindrance can be realized.

In particular, the endoscope information reading section is provided on the cleaning case for cleaning the accessories, which is disposed in substantially the center of the cleaning tank, and therefore, the operator can easily perform the operation of causing the endoscope information reading section to read the information of the endoscope to be set. Therefore, the merit of enhancing the operation efficiency, and the merit that the apparatus main body does not have to be made large can be provided.

Note that in the embodiment and the respective modifications described above, the endoscope information reading sections are ring-shaped, but the endoscope information reading sections do not have to be ring-shaped.

The present invention is not limited to the embodiment described above, and various modifications, alterations and the like can be made within the range without departing from the scope of the claims.

The present application is filed claiming the priority of Japanese Patent Application No. 2011-174279 filed in Japan on August 9, 2011.

## Claims

1. An endoscope cleaning/disinfecting apparatus (1), comprising:
a cleaning tank (4) capable of housing a plurality of endoscopes (100);
a position defining section (111) that is provided in the cleaning tank (4), and defines positions of the plurality of endoscopes (100) which are housed and set in the cleaning tank (4);
an endoscope information reading section (64) that is provided in the cleaning tank (4), and, in a state in which one endoscope (100) is set in the cleaning tank (4) in a position defined by the position defining section (111) so as to surround a central portion of the cleaning tank (4), is arranged in a site exposed toward an upper side of the central portion of the cleaning tank (4); and
an accessory cleaning section (6) that is disposed in the exposed site, and accommodates and cleans accessories of the plurality of endoscopes (100),
wherein the endoscope information reading section (64) is provided at the accessory cleaning section.

2. The endoscope cleaning/disinfecting apparatus (1) according to claim 1,
wherein the exposed site is a site that is exposed toward the upper side of the cleaning tank (4) without being covered even with the plurality of endoscopes (100) which are set in the cleaning tank (4) in positions defined by the position defining section (111).

3. The endoscope cleaning/disinfecting apparatus (1) according to claim 1,
wherein the accessory cleaning section is an accessory cleaning case (6) that is detachable with respect to the cleaning tank (4).

4. The endoscope cleaning/disinfecting apparatus (1) according to claim 3,
wherein the endoscope information reading section (64) has an antenna (65) for reading information of the respective endoscopes (100) by radio, and
the antenna (65) is provided at an upper side of the accessory cleaning case (6).

5. The endoscope cleaning/disinfecting apparatus (1) according to claim 4,
wherein the antenna (65) is provided in an inside of an edge portion (6a) of the upper side of the accessory cleaning case (6), or in a resin member (68) provided at the upper side.

6. The endoscope cleaning/disinfecting apparatus (1) according to claim 1,
wherein the accessory cleaning section has a concave portion (72) that accommodates the accessories, and
the endoscope information reading section (64) is provided at a top portion (71a) of a protruded portion (71) surrounding the concave portion (72).

7. The endoscope cleaning/disinfecting apparatus (1) according to claim 6,
wherein the endoscope information reading section (64B) is a resin member in which an antenna (65) for reading information of the respective endoscopes (100) by radio is embedded.

8. The endoscope cleaning/disinfecting apparatus (1) according to claim 1,
wherein the position defining section (111) is detachable with respect to the cleaning tank (4).

## Patentansprüche

1. Endoskopreinigungs/Desinfektionsgerät (1), das umfasst:
einen Reinigungstank (4), der dazu in der Lage ist, eine Mehrzahl von Endoskopen (100) aufzunehmen;
einen Positionsdefinitionsabschnitt (111), der in dem Reinigungstank (4) vorgesehen ist und der Positionen der Mehrzahl von Endoskopen (100), die in dem Reinigungstank (4) aufgenommen und in ihn eingesetzt sind, definiert;
einen Endoskopinformationsleseabschnitt (64), der in dem Reinigungstank (4) vorgesehen ist und in einem Zustand, in dem ein Endoskop (100) in den Reinigungstank (4) in einer Position eingesetzt ist, die durch den Positionsdefinitionsabschnitt (111) definiert ist, so dass es einen zentralen Abschnitt des Reinigungstank (4) umgibt, in einer Position angeordnet ist, die in Richtung einer oberen Seite des zentralen Abschnitts des Reinigungstank (4) exponiert ist; und
einen Zubehörreinigungsabschnitt (6),
der in der exponierten Position angeordnet ist und Zubehörteile der Mehrzahl von Endoskopen (100) aufnimmt und reinigt,
wobei der Endoskopinformationsleseabschnitt (64) an dem Zubehörreinigungsabschnitt vorgesehen ist.

2. Endoskopreinigungs/Desinfektionsgerät (1) gemäß Anspruch 1,
wobei die exponierte Position eine Position ist, die in Richtung der oberen Seite des Reinigungstank (4) exponiert ist ohne abgedeckt zu sein, selbst wenn die Mehrzahl von Endoskopen (100) in Positionen in den Reinigungstank (4) eingesetzt sind, die durch den Positionsdefinitionsabschnitt (111) definiert sind.

3. Endoskopreinigungs/Desinfektionsgerät (1) gemäß Anspruch 1,
wobei der Zubehörreinigungsabschnitt ein Zubehörreinigungsgehäuse (6) ist, das bezüglich des Reinigungstanks (4) lösbar ist.

4. Endoskopreinigungs/Desinfektionsgerät (1) gemäß Anspruch 3,
wobei der Endoskopinformationsleseabschnitt (64) eine Antenne (65) zum Lesen von Informationen der jeweiligen Endoskope (100) durch Funkübertragung hat und
die Antenne (65) an einer oberen Seite des Zubehörreinigungsgehäuses (6) vorgesehen ist.

5. Endoskopreinigungs/Desinfektionsgerät (1) gemäß Anspruch 4,
wobei die Antenne (65) in einem Inneren eines Kantenabschnitts (6a) der oberen Seite des Zubehörreinigungsgehäuses (6) oder in einem Harzelement (68), das an der oberen Seite vorgesehen ist, vorgesehen ist.

6. Endoskopreinigungs/Desinfektionsgerät (1) gemäß Anspruch 1,
wobei der Zubehörreinigungsabschnitt einen konkaven Abschnitt (72) hat, der die Zubehörteile aufnimmt, und
der Endoskopinformationsleseabschnitt (64) an einem oberen Abschnitt (71a) eines vorstehenden Abschnitts (41), der den konkaven Abschnitt (72) umgibt, vorgesehen ist.

7. Endoskopreinigungs/Desinfektionsgerät (1) gemäß Anspruch 6,
wobei der Endoskopinformationsleseabschnitt (64B) ein Harzelement ist, in dem eine Antenne (65) zum Lesen von Informationen der jeweiligen Endoskope (100) durch Funkübertragung eingebettet ist.

8. Endoskopreinigungs/Desinfektionsgerät (1) gemäß Anspruch 1,
wobei der Positionsdefinitionsabschnitt (111) bezüglich des Reinigungstanks (4) lösbar ist.

## Revendications

1. Appareil (1) de nettoyage/désinfection d'endoscopes, comprenant :
un réservoir de nettoyage (4) capable de loger une pluralité d'endoscopes (100) ;
une section (111) de définition de position qui est prévue dans le réservoir de nettoyage (4), et définit des positions de la pluralité d'endoscopes (100) qui sont logés et fixés dans le réservoir de nettoyage (4) ;
une section (64) de lecture d'informations d'endoscopes qui est prévue dans le réservoir de nettoyage (4), et, dans un état dans lequel un endoscope (100) est fixé dans le réservoir de nettoyage (4) dans une position définie par la section (111) de définition de position de façon à entourer une partie centrale du réservoir de nettoyage (4), est agencée au niveau d'un emplacement exposé vers un côté supérieur de la partie centrale du réservoir de nettoyage (4) ; et
une section (6) de nettoyage d'accessoires qui est disposée dans l'emplacement exposé, et reçoit et nettoie des accessoires de la pluralité d'endoscopes (100),
dans lequel la section (64) de lecture d'informations d'endoscopes est prévue au niveau de la section de nettoyage d'accessoires.

2. Appareil (1) de nettoyage/désinfection d'endoscopes selon la revendication 1,
dans lequel l'emplacement exposé est un emplacement qui est exposé vers le côté supérieur du réservoir de nettoyage (4) sans être couvert même avec la pluralité d'endoscopes (100) qui sont fixés dans le réservoir de nettoyage (4) dans des positions définies par la section (111) de définition de position.

3. Appareil (1) de nettoyage/désinfection d'endoscopes selon la revendication 1,
dans lequel la section de nettoyage d'accessoires est un boîtier (6) de nettoyage d'accessoires qui est détachable par rapport au réservoir de nettoyage (4).

4. Appareil (1) de nettoyage/désinfection d'endoscopes selon la revendication 3,
dans lequel la section (64) de lecture d'informations d'endoscopes comporte une antenne (65) destinée à lire des informations concernant les endoscopes respectifs (100) par radio, et
l'antenne (65) est prévue au niveau d'un côté supérieur du boîtier (6) de nettoyage d'accessoires.

5. Appareil (1) de nettoyage/désinfection d'endoscopes selon la revendication 4,
dans lequel l'antenne (65) est prévue à l'intérieur d'une partie de bord (6a) du côté supérieur du boîtier (6) de nettoyage d'accessoires, ou dans un élément en résine (68) prévu au niveau du côté supérieur.

6. Appareil (1) de nettoyage/désinfection d'endoscopes selon la revendication 1,
dans lequel la section de nettoyage d'accessoires comporte une partie concave (72) qui reçoit les accessoires, et
la section (64) de lecture d'informations d'endoscopes est prévue au niveau d'une partie supérieure (71a) d'une partie saillante (71) entourant la partie concave (72).

7. Appareil (1) de nettoyage/désinfection d'endoscopes selon la revendication 6,
dans lequel la section (64B) de lecture d'informations d'endoscopes est un élément en résine dans lequel une antenne (65) destinée à lire des informations concernant les endoscopes respectifs (100) par radio est intégrée.

8. Appareil (1) de nettoyage/désinfection d'endoscopes selon la revendication 1,
dans lequel la section (111) de définition de position est détachable par rapport au réservoir de nettoyage (4).
